# EUROPEAN PATENT APPLICATION

(11) **EP 2 762 873 A1**
(43) Date of publication of application: **06.08.2014**
(21) Application number: 12834964.4
(22) Date of filing: 26.09.2012
(51) Int. Cl.: G01N 27/416, G01N 27/327

(54) **METHOD FOR SPECIFICALLY DETECTING TEST SUBSTANCE**

(30) Priority: 26.09.2011 JP 2011209985
(71) Applicant: Toto Ltd., Fukuoka 802-8601 (JP)
(72) Inventor: BEKKI, Makoto, Fukuoka 802-8601 (JP); NARITA, Junya, Fukuoka 802-8601 (JP); OGAMI, Yumi, Fukuoka 802-8601 (JP); KANEHIRA, Koki, Fukuoka 802-8601 (JP)
(74) Representative: ABG Patentes, S.L.
(86) International application number: PCT/JP2012/074797
(87) International publication number: WO 2013/047624

(57) **Abstract**

In detection of a test substance including a biomolecule by utilizing photocurrent detection of dye sensitization, disclosed is a detection method in which process of the photocurrent detection is carried out by using an electrolyte solution not necessarily requiring an organic solvent. By making the electrolyte medium an aqueous system not containing an organic solvent, not only its usability can be enhanced but also measurement values with less dispersion can be obtained. Therefore, detection of the test substance including a biomolecule by utilizing the photocurrent detection of dye sensitization according to the present invention is characterized by that the processes from a reaction process of a test substance till detection of the photocurrent are carried out in a single apparatus, and that process of the photocurrent detection is carried out by using an electrolyte solution not necessarily requiring an aprotic solvent.

### [Sequence Listing]

SEQUENCE LISTING

<110>  TOTO Ltd.

<120>  Method for specifically detecting analyte

<130>  P10782EPPC

<140>  EP 12834964.4
<141>  2012-9-26

<150> JP2011-209985
<151> 2011-9-26

<160>  6

<170>  PatentIn version 3.5

<210>  1
<211>  19
<212>  DNA
<213>  Artificial Sequence

<220>
<223>  probe

<400>  1
tgtaggagct gctggtgca                                                    19


<210>  2
<211>  27
<212>  DNA
<213>  Artificial Sequence

<220>
<223>  primer

<400>  2
gatattgaac aatggttcac tgaagac                                           27


<210>  3
<211>  19
<212>  DNA
<213>  Artificial Sequence

<220>
<223>  primer

<400>  3
gcccctcagg gcaactgac                                                    19


<210>  4
<211>  250
<212>  DNA
<213>  Artificial Sequence

<220>
<223>  target

<400>  4

gatattgaac aatggttcac tgaagaccca ggtccagatg aagctcccag aatgccagag 60

gctgctcccc gcgtggcccc tgcaccagca gctcctacac cggcggcccc tgcaccagcc 120

ccctcctggc ccctgtcatc ttctgtccct tcccagaaaa cctaccaggg cagctacggt 180

ttccgtctgg gcttcttgca ttctgggaca gccaagtctg tgacttgcac ggtcagttgc 240

cctgaggggc 250

<210> 5
<211> 19
<212> DNA
<213> Artificial Sequence

<220>
<223> probe (SNP)

<400> 5
tgtaggagca gctggtgca 19

<210> 6
<211> 19
<212> DNA
<213> Artificial Sequence

<220>
<223> probe (MM)

<400> 6
tgagcaagtt cagcctggt 19

## Description

### TECHNICAL FIELD

The present invention relates to, in a method for specifically detecting by using a photocurrent a test substance having a specific binding property such as a nucleic acid, an exogenous endocrine disrupting chemical, and an antigen, a method with which the measurement condition thereof can be easily found as to whether it is appropriate or not; a sensor unit used for it; and a measurement instrument for it.

### BACKGROUND ART

The gene diagnostic method to detect and analyze a DNA in a biological sample is considered to be viable as a new protection method and a new diagnostic method of various diseases. To conveniently and accurately detect the DNA like this, following techniques have been proposed.

There has been known an analysis method of DNA in which a DNA to be tested is hybridized with a DNA probe having a base sequence complementary to this and also having been labeled with a fluorescent substance whereby detecting a fluorescent signal generated thereupon (see, for example, Patent Literature 1 and Patent Literature 2). In this method, formation of a double chain DNA by hybridization is detected by fluorescence of the dye. In this fluorescence detection, because plural detection spots fixed with the probe DNAs can be formed on the same plate, there is an advantage that specificities to the plural probe DNAs can be analyzed by single detection. However, the detection instrument including a light- acceptor unit necessary to detect the fluorescent light has a large foot print and is expensive, and in addition, unification of the double chain DNA into one chain, hybridization, washing, and fluorescence detection have to be done with respective different instruments; and thus, detection of DNA cannot be done conveniently.

Meanwhile, a solar cell with which an electric energy is generated from a light by using a sensitizing dye has been known (see, for example, Patent Literature 3). This solar cell has a polycrystalline metal oxide semiconductor and is formed of a sensitizing dye layer in the wide range of the surface area thereof.

As the attempt to make use of this characteristics of the solar cell to biological analysis, there has been a proposal to make use of the photocurrent generated by photoexcitation of the dye for detection of a test substance (biomolecules such as DNA and protein) (for example, Nakamura, et al., "A New Method for Detection of DNA Double Chain by Photoelectric Conversion", Proceeding of the Japan Chemical Society, Vol. 81 ST, No.2 (2002), p.947 (Non-Patent Literature 1)).

In addition, in the past, part of inventors of the present invention proposed a method, for example, the method proposed in Patent Literature 4, for specifically detecting by using a photocurrent a test substance having a specific binding property, such as a nucleic acid, an exogenous endocrine disrupting chemical, and an antigen.

In the past, for detection of photocurrent of dye sensitization including a solar cell, an electrolyte solution using acetonitrile solvent has been used. In detection of a test substance such as a biomolecule by making use of photocurrent detection of dye sensitization too, in the case that all the processes from a reaction of the test substance until detection of the photocurrent are carried out in a single apparatus, even though an aqueous solvent, a salt, and the like are used in the reaction process of the test substance, an electrolyte solution containing a conventional aprotic polar solvent such as acetonitrile has been used in detection of the photocurrent.
Patent Literature 1: JP H07-107999 A,
Patent Literature 2: JP H11-315095A,
Patent Literature 3: JP H01-220380 A,
Patent Literature 4: WO2007/037341 A
Non-Patent Literature 1: Surface Science, Vol. 24, No. 11, Pp. 671-676, 2003

### SUMMARY OF THE INVENTION

Inventors of the present invention found at this time that in detection of a test substance such as a biomolecule by making use of photocurrent detection based on the dye sensitization, in the case that the processes from the reaction of test substance till the detection of photocurrent were carried out in a single apparatus, in the photocurrent detection too, the test substance could be detected satisfactorily by using an aqueous electrolyte solution which is not containing an aprotic solvent, i.e., an organic solvent, containing an electrolyte capable of donating an electron to a protic solvent and a sensitizing dye. It was also found that by using an aqueous electrolyte solution as the electrolyte solution, the detection thereof could be done more stably. The present invention is based on these findings.

Accordingly, the present invention has, in detection of a test substance such as a biomolecule by making use of photocurrent detection based on the dye sensitization, an object to provide a method for detection thereof in which not only the processes from the reaction of the test substance till the detection of photocurrent are carried out in a single apparatus, but also an electrolyte solution not necessitating an aprotic solvent in the photocurrent detection process is used.

And thus, a method for specific detection of a test substance according to the present invention is a method which comprises the steps of contacting both a working electrode having a sensitizing dye-labeled test substance fixed thereto via a probe substance and a counter electrode with an electrolyte medium, and then irradiating the working electrode with light to cause photoexcitation of the sensitizing dye, whereby detecting a photocurrent flowing between the working electrode and the counter electrode caused by electron transfer from the photoexcited sensitizing dye to the working electrode,
wherein
the working electrode has an electron- acceptor layer formed by containing an electron- acceptor substance capable of accepting an electron that is discharged in response to photoexcitation of the sensitizing dye, and the probe substance is attached on surface of this electron- acceptor layer;
a binding reaction step for contacting the working electrode to the test substance in the presence of a reaction solution to obtain a working electrode having the sensitizing dye-labeled test substance fixed thereto via the probe substance,
a washing step after the binding reaction step for washing out unbound substances with a washing solution, and
a measurement step in which the electrolyte medium is charged, and then the light is irradiated whereby measuring a photocurrent thus formed, are carried out in a single sensor cell; and
the electrolyte medium contains water and an electrolyte capable of donating an electron to the sensitizing dye in the oxidized state, while does not substantially contain an aprotic solvent.

### BRIEF DESCRIPTION OF THE DRAWINGS

Fig. 1 shows an example of the apparatus for carrying out the detection method of the present invention.
Fig. 2 shows the sensor cell configuration for carrying out the detection method of the present invention.
Fig. 3 shows the configuration of the electrode unit of the sensor cell.
Fig. 4 shows the scatter diagram of the photocurrent values in each line in the cases of containing acetonitrile and not containing acetonitrile as the electrolyte medium.
Fig. 5 shows, by the bar charts, the average value of photocurrent values of the respective cases of using the perfect match probe (PM), the single nucleotide polymorphisms (SNP) probe, and the perfect mismatch probe (MM) in the detection method of the present invention.
Fig. 6 shows a plotting chart of the amounts of the sensitizing dye calculated from the fluorescent values relative to the photocurrent values in the corresponding spots in the detection method of the present invention.

### MODES FOR CARRYING OUT THE INVENTION

Specific detection of test substance by using photocurrent:
In the method of the present invention, firstly, a working electrode, a counter electrode, and a sample solution which contains a test substance are prepared. The working electrode to be used in the present invention is the electrode having on its surface a probe substance capable of forming a specific bind to the test substance directly or indirectly. That is, the probe substance may be not only the substance which forms a bind directly and specifically to the test substance but also the substance capable of forming a bind specifically to the complex which is obtained by specifically binding the test substance to a mediating substance such as a receptor protein molecule. Then, in the co-presence of the sensitizing dye, the sample solution is contacted to the working electrode to bind the test substance specifically to the probe substance directly or indirectly, whereby fixing the sensitizing dye to the working electrode by this bind. The sensitizing dye is a substance capable of releasing an electron to the working electrode in response to photoexcitation; and this may be labeled to the test substance or to the mediating substance in advance, or may be simply added to the sample solution in the case that the sensitizing dye capable of being intercalated into the complex of the test substance with the probe substance is used.

Then, after the working electrode and the counter electrode are contacted to the electrolyte medium, when a light is irradiated to the working electrode to cause photoexcitation of the sensitizing dye, an electron transfer takes place from the photoexcited sensitizing dye to the electron-acceptor substance present on surface of the working electrode. By detecting the photocurrent flowing between the working electrode and the counter electrode caused by this electron transfer, the test substance can be detected with high sensitivity. That is, it is thought that there is a relationship between the photocurrent value and amount of the sensitizing dye that is excited on the working electrode caused by the light irradiation. In addition, because it can be assumed that this detected photocurrent value also has a good relationship with amount of the test sample in the sample solution to which the sensitizing dye is bound with a certain ratio, the test sample may be quantitatively measured based on the measured amount of the current or the electric quantity.

The apparatus and the detection method according to the present invention based on the basic principle mentioned above are in the public domain; and in addition, there are the apparatus and the method filed for patent application by inventors including some inventors of the present invention. The apparatus and the detection method as mentioned above may be applied to the methods and the apparatuses described in the literatures which include: WO2007/037341, JP 2006-119111 A, JP 2006-119128 A, JP 2007-085941 A, JP 2008-020205 A, JP 2008-157915A, JP 2008-157916 A, JP 2008-192770 A, JP 2008-202995 A, JP 2009-186453 A, JP 2009-186454 A, JP 2009-186462 A, JP 2010-038813 A, and JP 2011-504882 A.

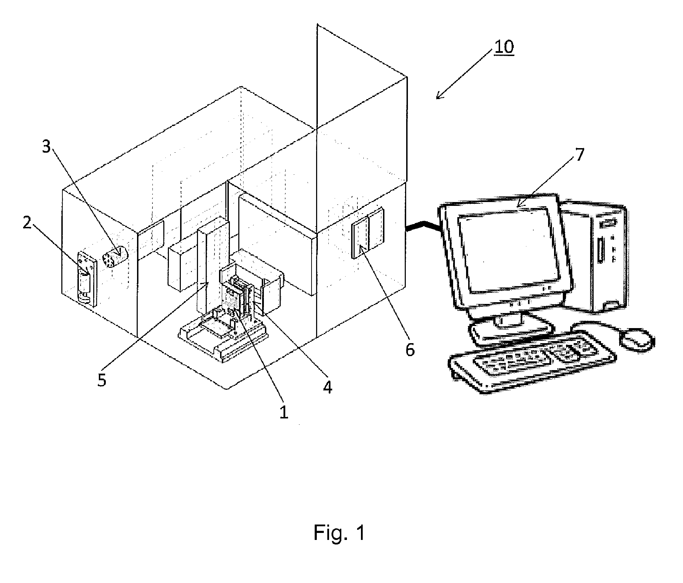

The basic features of the apparatus and the detection method according to the present invention based on the basic principle mentioned above will be explained by using the drawings. The measurement apparatus 10 shown in FIG. 1, which makes use of the photocurrent generated by photoexcitation of the dye for specific detection of the test substance, comprises, as the basic composition, the sensor cell 1, the fluid supply means 2 to supply the fluid to the sensor cell 1 and the switching hub 3 arranged therebetween, the light source laser head 4 to irradiate a light to the spots which attach the probe substance, the spots being arranged on the electron- acceptor layer of the working electrode installed inside the sensor cell 1, the XY automatic stage 5, and the temperature-controlling unit 6 arranged in the sensor cell to control the temperature-adjusting function during the time of reaction and detection; and this is connected to the personal computer 7 to process the signals sent from the measurement apparatus 1.

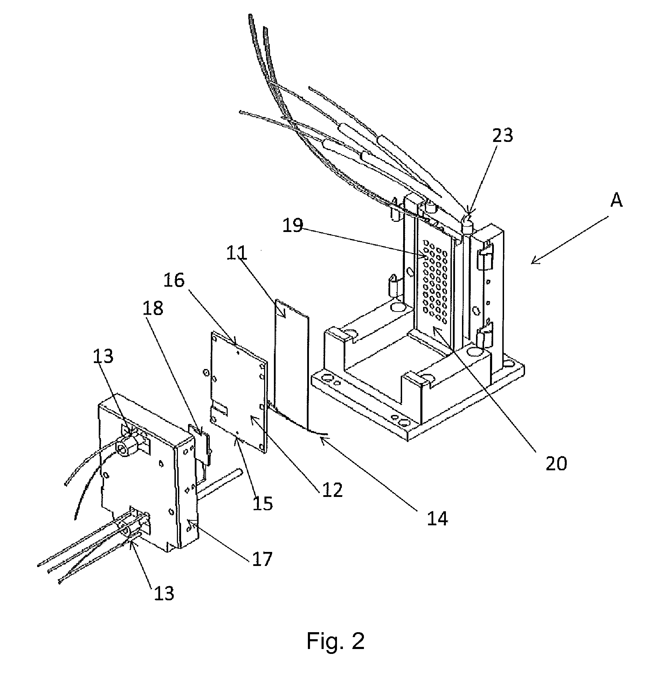

FIG. 2 is the drawing to show the structure of the sensor cell 1. In the sensor cell 1, the following processes can be carried out consistently: a binding reaction process in which at least contact of the working electrode to the test substance in the presence of a reaction solution is involved thereby obtaining a working electrode having the sensitizing dye-labeled test substance fixed thereto via the probe substance; a washing process in which a unbound substance after the binding reaction process is washed out by a washing solution; and a measurement process in which the electrolyte medium is charged, and then a light is irradiated, whereby measuring a photocurrent thus formed; and this sensor cell has one closed space structure formed by the construction components including the working electrode. Specifically, the sensor cell 1 has the working electrode 11, the counter electrode 12, and the working electrode conductive part 13 and the counter electrode conductive part 14 (these enable to conduct between the electrodes), wherein the counter electrode 12 has the inlet port 15 and the outlet port 16 through which the reaction solution, the washing solution, and the electrolyte medium are charged and discharged, respectively. The working electrode 11 and the counter electrode 12, together with the counter electrode heater 18, are fixed by the pressing part 17. A light is irradiated to the counter electrode 11 from the direction of A in Fig. 2, and this light goes to the working electrode 11 via plural openings 19 present in the positions corresponding to respective plural spots on the working electrode 11 and the pressing part 20 which has the working electrode heater 23. When the sensitizing dye is photoexcited, electron transfer occurs from the photoexcited sensitizing dye to the electron-acceptor substance (not shown in the drawing). By detecting the photocurrent flowing between the working electrode and the counter electrode caused by this electron transfer, the test substance can be detected with high sensitivity.

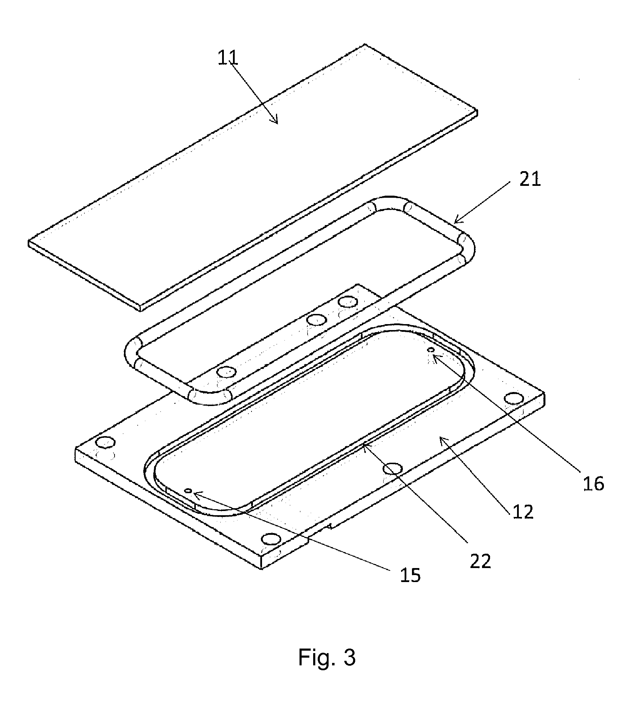

Fig. 3 is to explain further the structures of the working electrode 11 and the counter electrodes 12. Between the working electrode 11 and the counter electrode 12, the O-ring 21 to depart the both electrodes with a prescribed distance is put while setting into the depression portion 22 of the counter electrode. The inlet port 15 and the outlet port 16 are arranged at both edges inside the O-ring so that an air may be readily displaced with the solution inside the sensor cell.

Electrolyte medium in the photocurrent measurement process:
In the method according to the present invention, the following processes are carried out in the cell, namely, a binding reaction process in which at least contact of the working electrode to the test substance in the presence of a reaction solution is involved thereby obtaining a working electrode having the sensitizing dye-labeled test substance fixed thereto via the probe substance; a washing process in which a unbound substance after the binding reaction process is washed out by a washing solution; and a measurement process in which the electrolyte medium is charged, and then a light is irradiated whereby measuring a photocurrent thus formed. The present invention is characterized by that the electrolyte medium to be supplied in this measurement process contains water and an electrolyte capable of donating an electron to the sensitizing dye in the oxidized state, while not substantially containing an aprotic solvent. As a result of not containing the aprotic solvent, namely, substantially not containing an organic solvent, the electrolyte medium can be used conveniently; and in addition, this becomes an aqueous solvent system having less environmental burden due to the waste thereof. The aqueous medium may enhance usability thereof in the medical setting in which the method of the present invention will be actually used. In addition to enhancement of the usability thereof, it was found that stable measurement values, namely measurement values with less variation, could be obtained by changing the electrolyte medium to the aqueous system not containing the aprotic solvent. This can make one merit of using the aqueous solvent. It is not clear why the measurement with less dispersion became possible; but it is assumed that the effects of water and a salt introduced from the previous process may become rather small by using the aqueous solvent as the electrolyte medium.

According to the preferred embodiment of the present invention, when the salt concentration in the hybridization reaction solution as mentioned later is represented by A and the salt concentration in the washing solution as mentioned later is represented by B, ratio A/B satisfies the relationship of preferably 0.1≤A/B≤10 by mole ratio, more preferably 0.1≤A/B≤7.0 by mole ratio, or further more preferably 0.1≤A/B<7.0 by mole ratio. In addition, the salt concentration (A) in the hybridization reaction solution is preferably in the range of 0.05 to 1.2 mole/L, or more preferably in the range of 0.08 to 0.6 mole/L. The salt concentration (B) in the washing solution is preferably in the range of 0.05 to 1.2 mole/L, or more preferably in the range of 0.08 to 0.6 mole/L. Meanwhile, the respective salt concentrations are total salt concentrations by mole contained in the reaction solution and the washing solution, respectively. According to this embodiment, it was found that more stable measurement values, namely, measurement values with lesser dispersion could be obtained. According to the information obtained by the inventors of the present invention, it was observed that although the measurement values with less dispersion could be obtained stably by changing the electrolyte medium to the aqueous medium, the probe was occasionally released from the working electrode by changing to the aqueous medium. Release of the probe may cause a risk to give rise to dispersion of the measurement values in each spot. However, it is assumed that by arranging the salt concentration in the hybridization reaction solution and the salt concentration in the washing solution within the range that satisfies the relationships, this release of the probe can be suppressed, and as a result of it, the measurement values with less dispersion can be obtained. However, this explanation is just an assumption, so that the present invention is not restricted by this explanation.

The electrolyte contained in the electrolyte medium to be used in the present invention is not restricted, provided that it can move freely in the medium thereby being able to involve in giving and accepting an electron among the sensitizing dye, the working electrode, and the counter electrode; however, the electrolyte is preferably a substance which can function as a reducing agent (electron-donating agent) to give an electron to the dye which is excited by light irradiation. Illustrative example of the substance like this includes sodium iodide (Nal), potassium iodide (KI), calcium iodide (Cal2), lithium iodide (Lil), ammonium iodide (NH₄I), tetrapropyl ammonium iodide (NPr₄l), sodium thiosulfate (Na₂S₂O₃), sodium sulfite (Na₂SO₃), hydroquinone, ferricyanide salts such as potassium ferricyanide and ammonium ferricyanide, potassium ferrocyanide, K₄[Fe(CN)₆]·3H₂O (potassium ferrocyanide trihydrate), ammonium ferrocyanide, ammonium ferrocyanide trihydrate, ferrocene-1,1'-dicarboxylic acid, ferrocenecarboxylic acid, sodium borohydride (NaBH₄), triethylamine, ammonium thiocyanate, hydrazine (N₂H₄), acetaldehyde (CH₃CHO), N,N,N',N'-tetramethyl-p-phenylenediamine dihydrochloride salt (TMPD), L-ascorbic acid, sodium tellurite (Na₂TeO₃), ferric (II) chloride tetrahydrate (FeCl₂·4H₂O), EDTA, cysteine, triethanolamine, tripropylamine, iodine-containing lithium iodide (I/Lil), tris(2-chloroethyl) phosphate salt (TCEP), dithiothreitol (DTT), 2-mercaptoethanol, 2-mercaptoethanolamine, thiourea dioxide, (COOH)₂, HCHO, and a combination of them; more preferably potassium ferrocyanide, K₄[Fe(CN)₆]·3H₂O (potassium ferrocyanide trihydrate), ammonium ferrocyanide, ammonium ferrocyanide trihydrate, hydroquinone, L-ascorbic acid, Kl, and a mixture of them; and still more preferably potassium ferrocyanide.

In addition, the electrolyte medium may contain a substance which can function as a supporting electrolyte to lower solution resistance. Illustrative example of the substance like this includes chloride salts such as sodium chloride (NaCl), potassium chloride (KCI), lithium chloride (LiCl), rubidium chloride (RbCl), cesium chloride (ScCI), calcium chloride (CaCl₂), magnesium chloride (MgCl₂), and ammonium chloride (NH₄Cl); sulfate salts such as sodium sulfate (Na₂SO₄), potassium sulfate (K₂SO₄), lithium sulfate (Li₂SO₄), rubidium sulfate (Rb₂SO₄), cesium sulfate (Cs₂SO₄), calcium sulfate (CaSO₄), magnesium sulfate (MgSO₄), and ammonium sulfate ((NH₄)₂SO₄); nitrate salts such as sodium nitrate (NaNO₃), potassium nitrate (KNO₃), lithium nitrate (LiNO₃), rubidium nitrate (RbNO₃), cesium nitrate (ScNO₃), calcium nitrate (Ca(NO₃)₂), magnesium nitrate (Mg(NO₃)₂), and ammonium nitrate (NH₄NO₃); bromide salts such as sodium bromide (NaBr), potassium bromide (KBr), lithium bromide (LiBr), rubidium bromide (RbBr), cesium bromide (CsBr), calcium bromide (CaBr₂), magnesium bromide (MgBr₂), and ammonium bromide (NH₄Br); citrate salts such as monosodium citrate (NaH₂(C₆H₅O₇)), disodium citrate (Na₂H(C₆H₅O₇)), trisodium citrate (Na₃(C₆H₅O₇)), monopotassium citrate (KH₂(C₆H₅O₇)), dipotassium citrate (K₂H(C₆H₅O₇)), and tripotassium citrate (K₃(C₆H₅O₇)); acids and bases such as sulfuric acid (H₂SO₄), hydrochloric acid (HCl), sodium hydroxide (NaOH), and potassium hydroxide (KOH); biochemical buffer solutions such as Tris buffer, phosphate buffer, Good's buffers (ADA, PIPES, POPSO, ACES, MOPSO, BES, MOPS, TES, HEPES, DIPSO, TAPSO, POPSO, HEPPSO, EPPS, Tricine, Bicine, and TAPS), and a combination of them; more preferably sodium chloride (NaCl), potassium chloride (KCI), magnesium chloride (MgCl₂), sodium sulfate (Na₂SO₄), potassium sulfate (K₂SO₄), magnesium sulfate (MgSO₄), sodium nitrate (NaNO₃), potassium nitrate (KNO₃), magnesium nitrate (Mg(NO₃)₂), sodium bromide (NaBr), potassium bromide (KBr), magnesium bromide (MgBr₂), and a mixture of them; and further more preferably sodium chloride (NaCl).

According to the preferred embodiment of the present invention, when the salt concentration in the washing solution is represented by B and the salt concentration in the electrolyte medium is represented by C, ratio B/C satisfies the relationship of preferably 0.01≤B/C≤200 by mole ratio, more preferably 0.5≤B/C≤100 by mole ratio, or further more preferably 0.5≤B/C≤58 by mole ratio. In addition, the salt concentration (C) in the electrolyte medium is preferably in the range of 0.01 to 1.5 mole/L, or more preferably in the range of 0.01 to 0.9 mole/L. Meanwhile, the respective salt concentrations are total salt concentrations by mole contained in the washing solution and the electrolyte medium, respectively. According to this embodiment, it was found that more stable measurement values, namely, measurement values with lesser dispersion could be obtained. According to the information obtained by the inventors of the present invention, it was observed that although the measurement values with less dispersion could be obtained stably by changing the electrolyte medium to the aqueous medium, the probe was occasionally released from the working electrode by changing to the aqueous medium. Release of the probe may cause a risk to give rise to dispersion of the measurement values in each spot. However, it is assumed that by arranging the salt concentration in the washing solution and the salt concentration in the electrolyte medium within the range that satisfies the relationships, this release of the probe can be suppressed, and as a result of it, the measurement values with less dispersion can be obtained. It is also assumed that release of the specifically bound target DNA can be suppressed, thereby causing good effects as mentioned above. However, this explanation is just an assumption, so that the present invention is not restricted by this explanation.

Specific detection method of test substance:
As mentioned above, in the specific detection method of the test substance according to the present invention, the binding reaction process, the washing process, and the measurement process are carried out in a single cell, and in addition, the electrolyte medium used in the measurement process contains the electrolyte and water, but does not substantially contain an aprotic solvent; on the other hand, as to the conditions other than the above, those which are usually known or in the public domain in specific detection methods of the test substance may be used similarly.

To explain the general method thereof, for example, the hybridization reaction solution is charged into the sensor cell 1. Then, from the inlet port 15 arranged in the sensor cell 1, the test substance for the hybridization reaction is introduced thereinto; and then, the hybridization reaction with the probe substance, which is arranged on the working electrode and is capable of specifically binding to the test substance directly or indirectly, is executed in the sensor cell 1 whose temperature is adjusted to the prescribed temperature. Thereafter, the washing solution is charged thereinto to wash out the unbound substance.

In this washing process, it is necessary to remove only the unbound substance while the test substance bound to the probe substance remains to be bound as much as possible. Accordingly, it is preferable that the washing solution be blended with a surfactant. However, if an air bubble generated by charging and discharging of the washing solution remains on the probe arranged on the working electrode to react and detect on the working electrode in the sensor cell, there may be a chance to cause the unevenness in the reaction on surface of the working electrode; and thus, attention is necessary to generation and residue of the air bubble.

After washing, the washing solution is discharged from the sensor cell through the outlet port 16, and then, the electrolyte medium is charged thereinto; and thereafter, a light is irradiated from the light source into the sensor cell 1 to measure the current value caused by the photoexcitation. Composition of the electrolyte medium is as mentioned above. After the measurement, the electrolyte medium in the sensor cell 1 is discharged to outside the sensor cell.

According to the preferred embodiment of the present invention, the distance between the working electrode and the counter electrode is preferably in the range of 0.1 to 3 mm. As to the volume of the sensor cell, it is preferable that the hybridization reaction be done by using small amount of the test substance. According to this configuration, the hybridization reaction can be completed within a short period of time in the same sensor cell, and the photocurrent can be detected robustly.

### Hybridization reaction: reaction solution

In the detection method of the present invention, the reaction solution of the hybridization reaction is not particularly restricted, provided that the binding reaction of the test substance with the probe substance can take place therein, or is not disturbed thereby. According to the preferred embodiment of the present invention, this reaction solution contains a salt; and in addition, it is preferable that the salt concentration therein satisfy the above relationship with the salt concentration in the washing solution. Illustrative example of the salt contained in the reaction solution includes chloride salts such as sodium chloride (NaCl), potassium chloride (KCI), lithium chloride (LiCI), rubidium chloride (RbCl), cesium chloride (ScCI), calcium chloride (CaCl₂), magnesium chloride (MgCl₂), and ammonium chloride (NH₄Cl); sulfate salts such as sodium sulfate (Na₂SO₄), potassium sulfate (K₂SO₄), lithium sulfate (Li₂SO₄), rubidium sulfate (Rb₂SO₄), cesium sulfate (Cs₂SO₄), calcium sulfate (CaSO₄), magnesium sulfate (MgSO₄), and ammonium sulfate ((NH₄)₂SO₄); nitrate salts such as sodium nitrate (NaNO₃), potassium nitrate (KNO₃), lithium nitrate (LiNO₃), rubidium nitrate (RbNO₃), cesium nitrate (ScNO₃), calcium nitrate (Ca(NO₃)₂), magnesium nitrate (Mg(NO₃)₂), and ammonium nitrate (NH₄NO₃); bromide salts such as sodium bromide (NaBr), potassium bromide (KBr), lithium bromide (LiBr), rubidium bromide (RbBr), cesium bromide (CsBr), calcium bromide (CaBr₂), magnesium bromide (MgBr₂), and ammonium bromide (NH₄Br); citrate salts such as monosodium citrate (NaH₂(C₆H₅O₇)), disodium citrate (Na₂H(C₆H₅O₇)), trisodium citrate (Na₃(C₆H₅O₇)), monopotassium citrate (KH₂(C₆H₅O₇)), dipotassium citrate (K₂H(C₆H₅O₇)), and tripotassium citrate (K₃(C₆H₅O₇)); acids and bases such as sulfuric acid (H₂SO₄), hydrochloric acid (HCl), sodium hydroxide (NaOH), and potassium hydroxide (KOH); biochemical buffer solutions such as Tris buffer, phosphate buffer, Good's buffers (ADA, PIPES, POPSO, ACES, MOPSO, BES, MOPS, TES, HEPES, DIPSO, TAPSO, POPSO, HEPPSO, EPPS, Tricine, Bicine, and TAPS), and a combination of them; more preferably sodium chloride (NaCl), potassium chloride (KCI), magnesium chloride (MgCl₂), sodium sulfate (Na₂SO₄), potassium sulfate (K₂SO₄), magnesium sulfate (MgSO₄), sodium nitrate (NaNO₃), potassium nitrate (KNO₃), magnesium nitrate (Mg(NO₃)₂), sodium bromide (NaBr), potassium bromide (KBr), magnesium bromide (MgBr₂), trisodium citrate (Na₃(C₆H₅O₇)), and a mixture of them; and further more preferably sodium chloride (NaCl) and trisodium citrate (Na₃(C₆H₅O₇)).

### Washing solution:

According to the preferred embodiment of the present invention, it is preferable that after the hybridization process, the washing solution be charged for plural times to wash out the unbound substance thereby washing the spots. By so doing, the unbound substance can be removed so that only the test substances that are bound to the probe substance on the spots can remain.

According to the preferred embodiment of the present invention, this washing solution contains a salt; and in addition, it is preferable that the salt concentration therein satisfy the above relationship with the salt concentration in the reaction solution. Illustrative example of the salt contained in the washing solution includes chloride salts such as sodium chloride (NaCl), potassium chloride (KCI), lithium chloride (LiCI), rubidium chloride (RbCl), cesium chloride (ScCI), calcium chloride (CaCl₂), magnesium chloride (MgCl₂), and ammonium chloride (NH₄Cl); sulfate salts such as sodium sulfate (Na₂SO₄), potassium sulfate (K₂SO₄), lithium sulfate (Li₂SO₄), rubidium sulfate (Rb₂SO₄), cesium sulfate (Cs₂SO₄), calcium sulfate (CaSO₄), magnesium sulfate (MgSO₄), and ammonium sulfate ((NH₄)₂SO₄); nitrate salts such as sodium nitrate (NaNO₃), potassium nitrate (KNO₃), lithium nitrate (LiNO₃), rubidium nitrate (RbNO₃), cesium nitrate (ScNO₃), calcium nitrate (Ca(NO₃)₂), magnesium nitrate (Mg(NO₃)₂), and ammonium nitrate (NH₄NO₃); bromide salts such as sodium bromide (NaBr), potassium bromide (KBr), lithium bromide (LiBr), rubidium bromide (RbBr), cesium bromide (CsBr), calcium bromide (CaBr₂), magnesium bromide (MgBr₂), and ammonium bromide (NH₄Br); citrate salts such as monosodium citrate (NaH₂(C₆H₅O₇)), disodium citrate (Na₂H(C₆H₅O₇)), trisodium citrate (Na₃(C₆H₅O₇)), monopotassium citrate (KH₂(C₆H₅O₇)), dipotassium citrate (K₂H(C₆H₅O₇)), and tripotassium citrate (K₃(C₆H₅O₇)); acids and bases such as sulfuric acid (H₂SO₄), hydrochloric acid (HCl), sodium hydroxide (NaOH), and potassium hydroxide (KOH); biochemical buffer solutions such as Tris buffer, phosphate buffer, Good's buffers (ADA, PIPES, POPSO, ACES, MOPSO, BES, MOPS, TES, HEPES, DIPSO, TAPSO, POPSO, HEPPSO, EPPS, Tricine, Bicine, and TAPS), and a combination of them; more preferably sodium chloride (NaCl), potassium chloride (KCl), magnesium chloride (MgCl₂), sodium sulfate (Na₂SO₄), potassium sulfate (K₂SO₄), magnesium sulfate (MgSO₄), sodium nitrate (NaNO₃), potassium nitrate (KNO₃), magnesium nitrate (Mg(NO₃)₂), sodium bromide (NaBr), potassium bromide (KBr), magnesium bromide (MgBr₂), trisodium citrate (Na₃(C₆H₅O₇)), and a mixture of them; and further more preferably sodium chloride (NaCl) and trisodium citrate (Na₃(C₆H₅O₇)).

According to the preferred embodiment of the present invention, it is preferable that this washing solution contain a surfactant; and this surfactant is contained therein more preferably in the range of 0.001% to 10% (both ends inclusive). By blending the surfactant with the amount therein being 0.001 % or more, the washing efficiency can be enhanced. By using the surfactant with the amount therein being 10% or less, it becomes possible to suppress remaining of the air bubble generated by charging and discharging of the washing solution on the probe arranged on the working electrode to react and detect on the working electrode in the sensor cell, so that the unevenness in the reaction on surface of the working electrode can be suppressed.

According to the preferred embodiment of the present invention, it is preferable that the washing solution be charged for plural times to wash inside the sensor cell including the counter electrode also after the photocurrent detection process. Because inside the sensor cell can be washed after completion of the measurement, the next reaction and detection can be consecutively executed by exchanging only the working electrode without changing the counter electrode.

### Light source:

In the present invention, there is no restriction in the light source to be used, provided that it can irradiate a light having the wavelength at which the pilot dye can be photoexcited; and illustrative example thereof includes a fluorescent lamp, a black light, a sanitary lamp, an incandescent bulb, a low-pressure mercury lamp, a high-pressure mercury lamp, a xenon lamp, a mercury-xenon lamp, a halogen lamp, a metal halide lamp, LEDs (white, blue, green, and red), laser beams (CO2 laser, dye laser, and semiconductor laser), and a solar light. More preferable example thereof includes a fluorescent lamp, an incandescent bulb, a xenon lamp, a halogen lamp, a metal halide lamp, LEDs (white, blue, green, and red), and a solar light. In addition, as necessary, a light within a specific wavelength range may be irradiated by using a monochromator or a band pass filter.

According to the preferred embodiment of the present invention, in the case that plural kinds of test substances are detected separately by using two or more sensitizing dyes capable of being excited by respective different wavelengths, the plural dyes may be excited separately by irradiating the lights having specific wavelengths from the light source via a means to select the respective specific wavelengths. Illustrative example of the means to select the wavelengths includes a monochromator, a color glass filter, an intervening filter, and a band pass filter. In addition, plural light sources capable of irradiating the lights having respective different wavelengths in accordance with the kind of the sensitizing dye may be used; and in this case, the preferable light source includes a laser beam and an LED that can irradiate the light having specific wavelengths. In addition, in order to efficiently irradiate the light to the working electrode, the light may be guided by quartz, glass, or liquid guide.

### Test substance and probe substance:

There is no particular restriction in the test substance of the present invention, provided that this is a substance having a specific binding property; and thus, many kinds thereof may be used. If the test substance like this is used, by attaching a probe substance, which is capable of forming a specific bind to the test substance directly or indirectly, onto surface of the working electrode, the test substance may be detected by forming the specific bind to the probe substance directly or indirectly.

That is, in the present invention, the test substance and the probe substance that can form a specific bind therebetween may be selected. In other words, according to the preferred embodiment of the present invention, it is preferable that a substance which has a specific binding property be used as the test substance and a substance which forms a specific bind to the test substance be attached on the working electrode as the probe substance. By so doing, detection can be done by forming the specific bind with the test substance on the working electrode directly. As to the preferable examples of combination of the test substance with the probe substance in this embodiment, a combination of a single chain nucleic acid with a single chain nucleic acid which is complementary to the former, and a combination of an antigen with an antibody may be mentioned.

According to the more preferable embodiment of the present invention, it is preferable that a single chain nucleic acid be used as the test substance and a single chain nucleic acid which is complementary to the nucleic acid be used as the probe substance.

In the case that the single chain nucleic acid is used as the test substance, length of the base pair that constitutes the test substance is not restricted, provided that it has a complementary site to the nucleic acid that is the probe substance; however, it is preferable that the probe substance have 10 bp or more of the complementary site to the nucleic acid. According to the present invention, even if the nucleic acid has a relatively long chain having the base pairs of 200 bp, 500 bp, or 1000 bp, formation of the specific bind between the nucleic acids of both the probe substance and the test substance can be detected as the photocurrent with high sensitivity.

The sample solution containing the single chain nucleic acid as the test substance can be prepared by extracting the nucleic acid by a heretofore known method from various test samples containing a nucleic acid, including a blood such as a peripheral venous blood, a leukocyte, a serum, a urine, faces, a semen, a saliva, a cultured cell, and tissue cells such as various internal organ cells. During this process, destruction of the cells in the test sample may be done, for example, by vibrating the carrier by applying a physical impact such as vibration and ultrasonic wave from outside thereof. Alternatively, the nucleic acid may be extracted from the cell by using a nucleic acid extraction solution. Illustrative example of the nucleic acid eluting solution includes a solution containing saponin, EDTA, a protease, and a surfactant such as sodium dodecylsulfate (SDS), Triton-X, and Tween-20. In the case that the nucleic acid is eluted by using these solutions, the reaction may be facilitated by incubating the solution at the temperature of 37°C or higher.

According to the more preferred embodiment of the present invention, in the case that content of the genes used as the test substance is very small amount, it is preferable that the detection be carried out after the genes are amplified by a heretofore known method. The method for amplification of the genes may be typically represented by the method using an enzyme such as the enzyme responsible for polymerase chain reaction (PCR). Illustrative example of the enzyme used for the gene amplification method includes a DNA polymerase, a DNA-dependent DNA polymerase such as a Taq polymerase, a DNA-dependent RNA polymerase such as a RNA polymerase I, and a RNA-dependent RNA polymerase such as a Qβ replicase, while among them, the preferable method is the PCR method using a Taq polymerase because the amplification by it can be repeated continuously by only controlling the temperature thereat.

According to the preferred embodiment of the present invention, the nucleic acid can be specifically labeled with a sensitizing dye during the amplification mentioned above. Generally, the test substance labeled with a sensitizing dye may be obtained by amplifying the genes by using the PCR primer labeled with a sensitizing dye at the 5' terminal side thereof. Alternatively, the nucleic acid may be labeled with a sensitizing dye by incorporating thereinto a sensitizing dye-labeled dUTP or an aminoallyl-modified dUTP. In the case that the aminoallyl-modified dUTP is incorporated, in the next coupling stage, a sensitizing dye activated by N-hydroxysuccinimide undergoes a specific reaction with the modified dUTP to form the test substance that is uniformly labeled with the sensitizing dye.

According to the preferred embodiment of the present invention, a crude extraction solution of the nucleic acid obtained as mentioned above or a purified nucleic acid solution may be thermally denatured at 90 to 98°C, or preferably at 95°C or higher to obtain the single chain nucleic acid. Alternatively, the single chain nucleic acid may be prepared by carrying out the alkaline denaturation.

In the present invention, it may be allowed that the test substance and the probe substance form a specific bind indirectly. That is, according to another preferred embodiment of the present invention, it is preferable that by using a substance having a specific binding property as the test substance, in the co-presence of the substance which forms a specific bind with this test substance as a mediating substance, the substance capable of forming the specific bind with this mediating substance be supported on the working electrode as the probe substance. By so doing, even a substance which cannot form a specific bind to the probe substance may be detected by specifically binding onto the working electrode indirectly via the mediating substance. As to the preferable example of combination of the test substance, the mediating substance, and the probe substance, there may be mentioned a combination of a ligand, a receptor protein molecule capable of receiving this ligand, and a double chain nucleic acid capable of forming a specific bind to this receptor protein molecule. As to the preferable example of the ligand, an exogenous endocrine disrupting chemical (environment hormone) may be mentioned. The exogenous endocrine disrupting chemical is the substance which forms a bind to DNA via the receptor protein molecule thereby affecting the genetic expression to cause a harmful effect; and according to the method of the present invention, the binding property to DNA of the protein including the receptor provided by the test substance can be monitored conveniently.

According to the present invention, it is also possible that a plurality of the same test substances derived from different sources are reacted simultaneously with one probe substance thereby quantitatively analyzing the test substance derived from the intended source by judging the differences in amounts of the test substances from different sources. As to the specific application example thereof, the expression profile analysis by competitive hybridization on the microarray may be mentioned. In this method, to analyze difference in the expression pattern of a particular gene among cells, the test substances labeled with different fluorescent dyes are hybridized competitively to the same probe substance. In the present invention, by using the method like this, the expression difference analysis among cells can be done electrochemically, that is, the advantage not existed before can be obtained.

### Sensitizing dye:

In the present invention, in order to detect the presence of the test substance by the photocurrent, the test substance is bound specifically to the probe substance directly or indirectly in the co-existence of a sensitizing dye; and by means of this bind, the sensitizing dye is fixed to the working electrode. For this, in the present invention, the test substance or the mediating substance may be labeled with the sensitizing dye in advance. In the case that the sensitizing dye capable of being intercalated into the complex of the test substance with the probe substance (for example, the double chain nucleic acid after hybridization) is used, the sensitizing dye may be fixed to the probe substance by adding the sensitizing dye into the sample solution.

According to the preferred embodiment of the present invention, in the case that the test substance is a single chain nucleic acid, it is preferable that one sensitizing dye be labeled relative to one molecule of the test substance. In order to readily form the specific bind between the test substance and the probe substance, the labeling site in the single chain nucleic acid is preferably at either the 5' terminal or the 3' terminal of the single chain nucleic acid; and in order to make the labeling process more convenient, it is more preferable if the labeling be made at the 5' terminal of the test substance.

According to another preferred embodiment of the present invention, in order to increase the amount of the sensitizing dye held relative to one molecule of the test substance, it is preferable that two or more sensitizing dyes be labeled relative to one molecule of the test substance. By so doing, the amount of the held dye per unit specific surface area on the working electrode formed with the electron-acceptor substance thereon can be made larger, so that the photocurrent response can be observed more sensitively.

The sensitizing dye to be used in the present invention may be the substance which can release an electron to the working electrode in response to the photoexcitation, can transit to the photoexcited state by irradiation of the light from a light source, and can take the electron state such that an electron can be charged to the working electrode from the excited state. Accordingly, because it is sufficient only if the sensitizing dye to be used can take the electron state relative to the working electrode, especially to the electron-acceptor layer, various kinds of the sensitizing dye can be used, so that it is not necessary to use an expensive dye.

In the embodiment in which separate detections of plural test substances are made, it is sufficient only if the sensitizing dyes which label the respective test substances can be excited by the lights having respective different wavelengths, so that it is sufficient if the respective test substances can be excited separately, for example, by selecting the wavelength of the irradiating light. For example, if plural sensitizing dyes corresponding to plural test substances are used and the lights having different exciting wavelengths to respective sensitizing dyes are irradiated, the signals can be detected separately even if plural probes are present on the same spot. In the present invention, number of the test substances is not restricted; however, in considering the wavelength of the light irradiated from the light source and the absorbance characteristics of the sensitizing dye, 1 to 5 kinds thereof may be appropriate. The sensitizing dye usable in this embodiment only needs to be photoexcited within the wavelength region of the irradiating light, and thus, the maximum absorbance thereof is not necessarily within the said wavelength region. Meanwhile, whether or not the sensitizing dye has the photo-absorbing reaction at the particular wavelength may be measured by a UV- visible spectrophotometer (for example, by using UV-3150 manufactured by Shimadzu Corp.).

Specific example of the sensitizing dye includes metal complexes and organic dyes. Illustrative example of the preferable metal complex includes metal phthalocyanines such as copper phthalocyanine and titanyl phthalocyanine; a chlorophyll or its derivative; hemin; complexes of ruthenium, osmium, iron, and zinc, which are described in JP H01-220380 A and in JP H05-504023 A (for example, cis-dicyanate-bis(2,2'-bipyridyl-4,4'-dicarboxylate) ruthenium (II)); and an europium complex. Illustrative example of the preferable organic dye includes a metal-free phthalocyanine, a 9-phenyl xanthene dye, a cyanine dye, a metallocyanine dye, a carbocyanine dye, a xanthene dye, a triphenylmethane dye, an acridine dye, an oxazine dye, a coumarin dye, a merocyanine dye, a rhodacyanine dye, a polymethine dye, and an indigo dye. Other illustrative example of the preferable sensitizing dye includes Cy 3, Cy 3.5, Cy 5, Cy 5.5, Cy 7, Cy 7.5, and Cy 9 (all of them are manufactured by GE Healthcare Life Sciences, Inc.); Alexa Fluor 355, Alexa Fluor 405, Alexa Fluor 430, Alexa Fluor 488, Alexa Fluor 532, Alexa Fluor 546, Alexa Fluor 555, Alexa Fluor 568, Alexa Fluor 594, Alexa Fluor 633, Alexa Fluor 647, Alexa Fluor 660, Alexa Fluor 680, Alexa Fluor 700, and Alexa Fluor 750 (all of them are manufactured by Molecular Probes); and Dry 610, DY-615, DY-630, DY-631, DY-633, DY-635, DY-636, EVO blue 10, EVO blue 30, DY-647, DY-650, DY651, DYQ-660, and DYQ-661 (all of them are manufactured by Dyomics GmbH).

As to the preferable example of the sensitizing dye capable of intercalating into the double chain nucleic acid, acridine orange and ethidium bromide may be mentioned. When the sensitizing dyes like this are used, the double chain nucleic acid labeled with the sensitizing dye can be formed by merely adding it into the sample solution after hybridization of the nucleic acid; and thus, it is not necessary to label the single chain nucleic acid in advance.

### Working electrode and preparation thereof:

The working electrode to be used in the present invention is the electrode which is provided with the probe substance on surface thereof, and is capable of accepting an electron which is released as the sensitizing dye fixed thereto via the probe substance is photoexcited. Accordingly, provided that the electron transfer can take place with the sensitizing dye used therein, the configuration and the material of the working electrode are not restricted, and various configurations and materials may be allowed.

According to the preferred embodiment of the present invention, it is preferable that the working electrode have the electron-acceptor layer which contains an electron-acceptor substance capable of accepting an electron released in response to photoexcitation of the sensitizing dye, and be provided with a probe substance on surface of this electron-acceptor layer. In addition, according to the more preferred embodiment of the present invention, it is preferable that the working electrode be formed of further containing an electric conductive substrate, and the electron-acceptor layer be formed on this electric conductive substrate.

In the present invention, the electron-acceptor layer contains an electron-acceptor substance capable of accepting an electron that is released as the sensitizing dye fixed thereto via the probe substance is photoexcited. That is, the electron-acceptor substance may be the substance that can take an energy level capable of being charged with an electron from the photoexcited pilot dye. Meanwhile, the energy level (A) capable of being charged with an electron from the photoexcited pilot dye means, for example, the conduction band (CB), if a semiconductor is used as the electron-acceptor material. That is, the electron-acceptor substance to be used in the present invention may be the substance having this level A, the level lower than the LUMO energy level of the sensitizing dye, in other words, the substance having the energy level lower than the LUMO energy level of the sensitizing dye may be used.

According to the preferred embodiment of the present invention, the electron-acceptor substance is preferably at least one kind selected from the group consisting of titanium oxide, zinc oxide, tin oxide, niobium oxide, strontium titanate, indium oxide, indium-tin composite oxide (ITO), and fluorine-doped tin oxide (FTO). Most preferably, TiO₂, indium-tin composite oxide (ITO), or fluorine-doped tin oxide (FTO) may be used. ITO and FTO have the functional properties not only as the electron-acceptor layer but also as the electric conductive substrate; and thus, by using these materials, the functions as the working electrode may be expressed only by the electron-acceptor layer without using the electric conductive substrate.

When a semiconductor is used as the electron-acceptor substance, this semiconductor may be any of single crystal and polycrystalline body; however, a polycrystalline body is preferable; and in addition, a porous semiconductor is more preferable than a compact one. Because the specific surface area becomes larger by so doing, the test substance and the sensitizing dye can be adsorbed much more, so that the test substance can be detected with much higher sensitivity. Therefore, according to the preferred embodiment of the present invention, the electron-acceptor layer is porous with each pore diameter being preferably in the range of 3 to 1000 nm or more preferably in the range of 10 to 100 nm.

According to the preferred embodiment of the present invention, the surface area in the state that the electron-acceptor layer is formed on the electric conductive substrate is preferably 10 times or more, or more preferably 100 times or more, relative to the projected area thereof. The upper limit of this surface area is not particularly restricted; but it may be usually about 1000 times. The particle diameter of fine particles to constitute the electron-acceptor layer, in terms of the average particle diameter of the primary particle by using the diameter when the projected area is converted to a circle, is preferably in the range of 5 to 200 nm, more preferably in the range of 8 to 100 nm, or still more preferably in the range of 20 to 60 nm. In addition, the average diameter of the fine particles (secondary particles) of the electron-acceptor substance in dispersion is preferably in the range of 0.01 to 100 µm. Alternatively, in order to enhance the light-capturing rate by scattering the incident light, the electron-acceptor layer may be formed by concurrently using the fine particles of the electron-acceptor substance whose particle size is large, for example, about 300 nm.

By taking the concavo-convex structure, surface area of the electron-acceptor layer can be made larger, so that more probe molecules can be fixed thereto, whereby the detection sensitivity can be enhanced. Because the sizes of biomolecules are in the range of about 0.1 nm to about 20 nm, the pour diameter formed by the concavo-convex structure is preferably in the range of 20 to 150 nm (both ends inclusive). If the gate to the space formed by the concavo-convex structure is narrower than this, the biomolecules cannot bind to the probe even though the specific surface area becomes larger, so that the detection signal becomes lower. On the other hand, if the concavo-convex structure is too rough, surface area does not increase so much that the signal strength may not increase so much. More preferable range for sensing of biomolecules is in the range of 50 to 150 nm (both ends inclusive).

According to the preferred embodiment of the present invention, it is preferable to adopt the pillar structure as the convex structure wherein pillars with nanometer sizes are arranged regularly on surface thereof. Although many methods have been known to make this structure, a method in which an anodized alumina having pores of nanometer size is used as the template is generally used. A method is known in which after a ceramic sol is charged into the template and heat-treated, the alumina template thereof is removed by etching; and another method is known in which after a charged ceramic sol is removed from the template, heat-treatment thereof is carried out. As to the method to make the nanostructure having a pillar shape, a method in which the nanostructure is made on a transparent substrate or a transparent conductive layer may be mentioned, as shown in JP 2004-130171 A. In this method, a titania sol is charged into the template of the anodized alumina, heated at 300 to 400°C, and then the template is removed by etching. As a result of it, a nanotube and a nanowire formed of titania are formed as the nanostructure.

According to the preferred embodiment of the present invention, it is preferable to adopt the air cavity as the convex structure which is formed because an organic substance becomes a gas phase by oxidative decomposition by burning an inorganic-organic hybrid precursor which contains a ceramic component. The inorganic-organic hybrid precursor is the one in which a metal-oxygen network structure, an organic polymer, and the like coexist, wherein the metal-oxygen network structure is formed by oxidation of an organometallic compound (metal alkoxide) followed by a polycondensation thereof. Alternatively, there is also a method in which an organic polymer or the like is added to commercially available titanium oxide particles (for example, the anatase-type crystal AMT-600 (trade name, average particle diameter of 30 nm) manufactured by TAYCA Corp.) or to a titanium oxide disperse solution. Many proposals have been made as to the composition thereof; for example, in JP H10-212120 A, a composition is proposed that is obtained by dispersing titanium oxide particles, together with an organic polymer as a dispersing adjuvant, in a glyme solvent ((HO-(-CH₂CH₂O-)ₙ-R; n represents 1 to 10, and R represents an alkyl group or an aryl group). When the disperse solution having this composition was applied on a carrier by an appropriate method (dip coating method, spray coating method, spinner coating method, blade coating method, roller coating method, wiper bar coating method, and reverse roll coating method) and then burnt at 200 to 800°C, specific surface area of 40 to 50 cm² per 1 cm² (thickness of 1 µm) could be achieved. Alternatively, a method is disclosed in JP 2001-233615 A in that a sol solution comprising a tetraalkoxytitanium, a stabilizer, a solvent, and ethylene oxide-propylene oxide-ethylene oxide block copolymer is dropped onto a base plate; the solvent is evaporated by rotating the base plate at high rotation speed thereby turning it to a gel to give an organic-inorganic composite titania thin film having a three-dimensional structure; and then, this is burnt at high temperature to remove the block copolymer, whereby achieving the fine three-dimensional convex structure. In addition, a method in which an oligosaccharide (trehalose) is used as the organic polymer thereby giving a ceramic porous film having porosity of 38 to 56% is disclosed (JP 2004-83376 A).

As mentioned above, many methods have been proposed regarding control of the fine concavo-convex structure of ceramics; and thus, by applying these methods appropriately to the ceramic electrode material of the present invention, the electrode material having a large specific surface area may be obtained.

According to the preferred embodiment of the present invention, it is preferable that the working electrode contain further an electric conductive substrate, and that the electron-acceptor layer be formed on the electric conductive substrate. The electric conductive substrate usable in the present invention may be not only those having electrical conductivity in the carrier itself such as titanium and other metals but also those having an electric conductive material layer on surface of a glass or a plastic carrier. In the case that this electric conductive substrate having the electric conductive material layer is used, the electron-acceptor layer is formed on the electric conductive material layer. Illustrative example of the electric conductive material to constitute the electric conductive material layer includes metals such as platinum, gold, silver, copper, aluminum, rhodium, and indium; electric conductive ceramics such as carbon, carbon carbide, and nitride; and electric conductive metal oxides such as indium-tin composite oxide, fluorine-doped tin oxide, antimony-doped tin oxide, gallium-doped zinc oxide, and aluminum-doped zinc oxide; while indium-tin composite oxide (ITO) and fluorine-doped tin oxide (FTO) are more preferable. However, as mentioned before, if the electron-acceptor layer itself can function also as the electric conductive substrate, the electric conductive substrate may be omitted. In addition, in the present invention, the electric conductive substrate is not restricted, provided that the material thereof can secure the electrical conductivity, so that it includes the electric conductive material layer in the form of a thin film or of a spot, not having strength by itself.

According to the preferred embodiment of the present invention, it is preferable that the electric conductive substrate be substantially transparent; specifically, the light-transmittance thereof is preferably 10% or more, more preferably 50% or more, or still more preferably 70% or more. By so doing, the cell can be made to have a configuration such that the light irradiated from the backside of the working electrode (namely the electric conductive substrate) and transmitted through the working electrode (namely the electric conductive substrate and the electron-acceptor layer) may excite the sensitizing dye. In addition, according to the preferred embodiment of the present invention, thickness of the electric conductive material layer is preferably in the range of about 0.02 to about 10 µm. Further, according to the preferred embodiment of the present invention, surface resistivity of the electric conductive substrate is 100Ω/cm² or less, or more preferably 40 Ω/cm² or less. The lower limit of the surface resistivity of the electric conductive substrate is not particularly restricted; but it may be usually about 0.1 Ω/cm².

Illustrative example of the preferable method for forming the electron-acceptor layer on the electric conductive substrate includes a method in which a disperse solution or a colloid solution of the electron-acceptor substance is applied onto the electric conductive carrier, a method in which a precursor of semiconductor fine particles is applied onto the electric conductive carrier, which is followed by hydrolysis thereof to obtain a fine particle film (sol-gel method), a sputtering method, a CVD method, a PVD method, a vapor-depositing method, and a plating method. Illustrative example of the method for forming the disperse solution of the semiconductor fine particles as the electron-acceptor substance includes, in addition to the foregoing sol-gel method, a method in which a mortar is used for grinding, a method in which dispersion is formed while grinding by using a mill, and a method in which semiconductor fine particles are used as they are, when separated in a solvent during synthesis thereof. Illustrative example of the dispersing medium used therein includes water and various organic solvents (for example, methanol, ethanol, isopropyl alcohol, dichloromethane, acetone, acetonitrile, and ethyl acetate). During dispersion operation, a polymer, a surfactant, an acid, a chelating agent, or the like may be used as a dispersing adjuvant, if necessary.

Illustrative example of the preferable application method of the disperse solution or of the colloid solution of the electron-acceptor substance includes an application type method such as a roller method and a dipping method; a metering type method such as an air knife method and a blade method; a method, in which application and metering can be done in the same part, such as a wire bar method disclosed in JP S58-4589 A; a slide hopper method disclosed in U. S. Patent 2681294, U. S. Patent 2761419, and U. S. Patent 2761791; and an extrusion method, a curtain method, a spinning method, and a spray method.

According to the preferred embodiment of the present invention, in the case that the electron-acceptor layer is formed of the semiconductor fine particles, thickness of the electron-acceptor layer is preferably in the range of 0.1 to 200 µm, more preferably in the range of 0.1 to 100 µm, still more preferably in the range of 1 to 30 µm, or utmost preferably in the range of 2 to 25 µm. By so doing, amounts of the probe substance and of the sensitizing dye to be fixed per unit projection area may be increased; and thus, not only the photocurrent may be increased but also loss of the generated electron due to re-binding of the electric charges may be decreased. In addition, coating amount of the semiconductor fine particles per 1 m² of the electric conductive substrate is preferably in the range of 0.5 to 400 g, or more preferably in the range of 5 to 100 g.

According to the preferred embodiment of the present invention, in the case that the electron-acceptor substance is formed of containing indium-tin composite oxide (ITO) or fluorine-doped tin oxide (FTO), thickness of the electron-acceptor layer is preferably 1 nm or more, or more preferably in the range of 10 nm to 1 µm.

According to the preferred embodiment of the present invention, it is preferable that heat-treatment be done after application of the semiconductor fine particles onto the electric conductive substrate. By so doing, particles can be contacted electrically among themselves, and in addition, strength of the coat film as well as adhesion with the carrier may be enhanced. The heat-treatment temperature is preferably in the range of 40 to 700°C, or more preferably in the range of 100 to 600°C. In addition, the time for the heat-treatment is in the range of about 10 minutes to about 10 hours.

In addition, according to another preferred embodiment of the present invention, in the case that the electric conductive substrate having a low melting point and a low softening temperature, such as a polymer film, is used, in order to prevent thermal deterioration thereof from occurring, it is preferable that film formation be done by using a method not using a high-temperature process; and illustrative example of the film formation method like this includes a press method, a low-temperature heating method, an electron beam irradiation method, a microwave irradiation method, an electrophoresis method, a sputtering method, a CVD method, a PVD method, and a vapor-deposition method.

The probe substance is attached on surface of the electron-acceptor layer of the working electrode thus obtained. Attachment of the probe substance to the working electrode may be done by a heretofore known method. According to the preferred embodiment of the present invention, in the case that a single chain nucleic acid is used as the probe substance, an oxide layer is formed on surface of the working electrode, and then, the nucleic acid probe and the working electrode can be bonded via this oxide layer. At this time, the nucleic acid probe can be fixed to the working electrode by introducing a functional group to terminal of the nucleic acid. By so doing, the nucleic acid probe having the functional group introduced therein can be fixed by the fixation reaction onto the carrier as it is. Introduction of the functional group to the nucleic acid terminal may be done by an enzymatic reaction or by using a DNA synthesizing apparatus. Illustrative example of the enzyme to be used in the enzymatic reaction includes terminal deoxynucleotidyl transferase, poly A polymerase, polynucleotide kinase, DNA polymerase, polynucleotide adenyl transferase, and RNA ligase. Alternatively, the functional group may be introduced by a polymerase chain reaction method (PCR method), a nick translation method, or a random primer method. The functional group may be introduced at any site of the nucleic acid, namely, at the 3' terminal site, at the 5' terminal site, or at random sites.

According to the preferred embodiment of the present invention, illustrative example of the preferable functional group to be used for fixation of the nucleic acid probe to the working electrode includes an amine, a carboxylic acid, a sulfonic acid, a thiol, a hydroxy group, and a phosphoric acid. According to the preferred embodiment of the present invention, in order to firmly fix the nucleic acid probe to the working electrode, a material that can crosslink between the working electrode and the nucleic acid probe may also be used. Illustrative example of the preferable crosslinking material includes a silane coupling agent, a titanate coupling agent, and electric conductive polymers such as polythiophene, polyacetylene, polypyrrole, and polyaniline.

According to the preferred embodiment of the present invention, fixation of the nucleic acid probe may also be done efficiently by more convenient process called physical adsorption. Physical adsorption of the nucleic acid probe to the electrode surface may be done, for example, as following. Firstly, the electrode surface is cleaned by distilled water and alcohol by using an ultrasonic cleaning machine. Then, the electrode is soaked in a buffer solution containing the nucleic acid probe to adsorb the nucleic acid probe onto the carrier surface.

After adsorption of the nucleic acid probe, non-specific adsorption may be suppressed by adding a blocking agent thereto. There is no particular restriction in the usable blocking agent, provided that it can cover the site not adsorbed with the nucleic acid probe on surface of the electron-acceptor layer and that it can be adsorbed to the electron-acceptor substance by chemical adsorption, physical adsorption, or the like; however, preferable example thereof is the substance having a functional group capable of being adsorbed via chemical bonding. For example, when titanium oxide is used as the electron-acceptor layer, illustrative example of the preferable blocking agent includes substances having functional groups that are capable of being adsorbed to titanium oxide, such as a carboxylic acid group, a phosphoric acid group, a sulfonic acid group, a hydroxyl group, an amino group, a pyridyl group, and amide group.

According to the preferred embodiment of the present invention, it is preferable that the probe substance be attached to each of plural separated areas on the working electrode, and that light irradiation from the light source be done separately to each area. By so doing, plural samples may be measured on one sheet of the working electrode; and thus, integration and the like of the DNA chips may become possible. According to the more preferred embodiment of the present invention, it is preferable that a pattern be formed such that plural areas having the probe substance attached onto the working electrode may be separated with each other, and that the test substance of the samples in each area may be continuously detected or determined quantitatively by one operation while scanning by a light irradiated from the light source.

According to the more preferred embodiment of the present invention, plural kinds of the probe substances may be attached in each area of plural areas which are separated with each other on the working electrode. By so doing, many samples, number of which is obtained by multiplying number of areas by number of the probe substances in each area, may be measured simultaneously.

According to the more preferred embodiment of the present invention, different probe substances may be attached in each area of plural areas which are separated with each other on the working electrode. By so doing, probe substances with number of the kind thereof corresponding to number of the separated area can be attached; and thus, many kinds of test substances may be measured simultaneously.

### Counter electrode:

The counter electrode to be used in the present invention is not particularly restricted, provided that a current can flow with the working electrode when it is contacted to the electrolyte medium; and thus, a carrier such as a glass, a plastics, a ceramics, and SUS, while these being vapor-deposited with a metal or an electric conductive oxide, may be used. Alternatively, a metal thin film may be formed as the counter electrode such that thickness thereof may be 5 µm or less, or preferably in the range of 3 nm to 3 µm by a method such as a vapor-deposition method and a sputtering method. Illustrative example of the preferable material usable for the counter electrode includes platinum, gold, palladium, nickel, carbon, an electric conductive polymer such as polythiophene, and electric conductive ceramics such as an oxide, a carbide, and a nitride; while platinum and carbon are more preferable; and further, platinum is the utmost preferable. These materials may be formed into a thin film by the methods similar to those used for formation of the electron-acceptor layer.

### EXAMPLES

The present invention will be explained in more detail by the following Examples; but the present invention is not limited to these Examples.

### Example 1

### Comparison by the electrolyte medium:

By using the method of the present invention, the p53 gene was detected while changing the detection condition of the electrolyte medium for comparison.

The perfect match probe DNA was fixed as the probe DNA on the working electrode side. Base sequences shown below were used.
Perfect match probe: 5'-TGTAGGAGCTGCTGGTGCA-3' (SEQ ID No. 1)

The perfect match target DNA to hybridize with this probe DNA was amplified by the PCR method. The base sequences of the primers and of the template DNA used in amplification of the perfect match target DNA by PCR are respectively shown below.
Forward primer: 5'-Cy5-GATATTGAACAATGGTTCACTGAAGAC-3' (SEQ ID No. 2)
Reverse primer: 5'-GCCCCTCAGGGCAACTGAC-3' (SEQ ID No. 3)

Because multiplication is done by using the forward primer whose 5' terminal side is labeled with Cy5, one molecule of the Cy5 dye is labeled relative to one molecule of the target DNA.
Template DNA: 5'-GATATTGAACAATGGTTCACTGAAGACCCAGGTCCAGATGAAGCTCCCAGA ATGCCAGAGGCTGCTCCCCGCGTGGCCCCTGCACCAGCAGCTCCTACACC GGCGGCCCCTGCACCAGCCCCCTCCTGGCCCCTGTCATCTTCTGTCCCTT CCCAGAAAACCTACCAGGGCAGCTACGGTTTCCGTCTGGGCTTCTTGCATT CTGGGACAGCCAAGTCTGTGACTTGCACGGTCAGTTGCCCTGAGGGGC-3' (SEQ ID No. 4)

The coat glass (U film with the sheet resistivity of 12 Ω/square and the shape of 75 mm x 25 mm, manufactured by AGC Fabritech Co., Ltd.) having the fluorine-doped tin oxide (F-SnO₂: FTO) was arranged as the glass substrate for the working electrode. This glass substrate was cleaned by soaking in acetone for 15 minutes, and then by ultrapure water in the ultrasonic bath for 15 minutes to remove a dirt and an organic residue. This glass substrate was shaken in a 0.5-M aqueous sodium hydroxide solution for 15 minutes. Thereafter, to remove sodium hydroxide, it was shaken for cleaning in ultrapure water for 5 minutes; and this operation was repeated for 3 times by changing the water. The glass substrate was taken out, and after the residual water was blown off by air, the glass substrate was soaked in anhydrous methanol to remove water. 3-Aminopropyl trimethoxy silane (manufactured by Alfa Aesar, A Johnson Matthey Company) was added to a 95% by volume methanol aqueous solution such that the concentration thereof may become 0.2% by volume; and then, the mixture was stirred at room temperature for 5 minutes to obtain a coupling-treatment solution. The glass substrate was soaked in this coupling-treatment solution, and then, it was shaken slowly for 15 minutes.

Next, the glass substrate was taken out and then shaken in methanol for 3 minutes; and this operation was repeated for 3 times by changing methanol to carry out the operation to remove the excess coupling-treatment solution. Thereafter, the glass substrate was kept at 110°C for 30 minutes to bond the coupling agent to the glass substrate. After the glass substrate was cooled to room temperature, the probe DNA with the concentration of 10 µM was prepared; and then, after it was kept at 95°C for 10 minutes, it was immediately transferred onto an ice and kept there for 10 minutes to denature the DNA. This probe DNA after denaturation was spotted in 10 lines in the long side direction and 2 rows in the short side direction with total 20 spots on the working electrode with the distance between the spot centers being 5 mm by using the Microarrayer LT-BA (spot pin diameter of 1.5 mm, manufactured by Filgen, Inc.). Thereafter, the solvent was evaporated by keeping it at 95°C for 3 minutes, and then a UV light of 120 mJ was irradiated by using the UV Crosslinker CL-1000 (manufactured by UVP, LLC) to fix the probe DNA to the glass substrate. Subsequently, cleaning thereof was repeated for 3 times by shaking it in an aqueous solution of 0.2 % by weight/volume of sodium dodecylsulfate (SDS, manufactured by Wako Pure Chemical Industries, Ltd.); and then, further cleaning thereof in ultrapure water by shaking for 5 minutes was repeated for 3 times. After this glass substrate was soaked in boiling water for 2 minutes and taken out therefrom, residual water was blown off by air. Then, the glass substrate was soaked in anhydrous ethanol at 4°C for 1 minute to remove water, and then residual ethanol was blown off by air. By so doing, the working electrode fixed with the probe DNA was obtained.

This electrode fixed with the probe DNA was installed as the working electrode in the sensor cell 1 of the apparatus shown in Fig.1. Installation thereof was done as shown Fig. 2 in such a way that the working electrode 11 was attached with the spacer having 0.5 mm thickness (not shown in the drawing) and disposed in a position so as to face to the counter electrode 12. An electrode was used as the counter electrode. This sensor cell has a flow cell structure having a flow channel which is formed by the spacer and the both electrodes. This has the fluid inlet port and the fluid outlet port, the both being connected to this flow channel in the opposite ends. To the fluid inlet port is connected the piping tube which is connected to the pump to charge the fluid into the sensor cell; and to the fluid outlet port is connected the piping tube to discharge the fluid which is charged into the sensor cell.

Next, 100 µL of an aqueous solution of the perfect match target DNA with the concentration thereof being adjusted 200 nM was mixed with 100 µL of an aqueous alkaline denaturing solution (2 mM of ethylenediamine tetraacetic acid (EDTA, manufactured by Wako Pure Chemical Industries, Ltd.) and 0.2 M of sodium hydroxide (NaOH, manufactured by Wako Pure Chemical Industries, Ltd.)); and then, the resulting mixture solution was kept at room temperature for 5 minutes to alkaline-denature the double chain DNA to the single chain DNA. To these solutions were added 700 µL of a dilute aqueous hybridization solution (5 x SSC, 0.1% SDS) and 100 µL of an aqueous neutralization solution (0.2 M hydrochloric acid (HCl, manufactured by Wako Pure Chemical Industries, Ltd.)), while temperatures of these solutions were adjusted at the prescribed hybridization temperatures; and then, they were mixed. This solution (1 mL) after mixing was charged into the sensor cell, and then kept at 45°C in the sensor cell for 10 minutes to carry out the hybridization with the probe DNA fixed to the working electrode. In this occasion, before the hybridization solution was charged into the sensor cell, temperature inside the sensor cell was adjusted at the prescribed hybridization temperature by using the temperature controlling member comprised of a ceramic heater, a thermostat, and a thermistor. Ten minutes thereafter, the hybridization solution was completely discharged from inside the sensor cell by charging an air by using a pump. Then, to wash out the excess target DNA and so forth not used for the hybridization, 1500 µL of an aqueous washing solution (3.5 x SSC) was charged into the sensor cell. Meanwhile, with regard to SSC, 20 x SSC means the SSC solution of 20-fold concentration; and composition of 20 x SSC is an aqueous mixture solution of 0.3 M sodium citrate (manufactured by Wako Pure Chemical Industries, Ltd.) and 3 M of sodium chloride (manufactured by Wako Pure Chemical Industries, Ltd.) (the same shall apply hereafter).

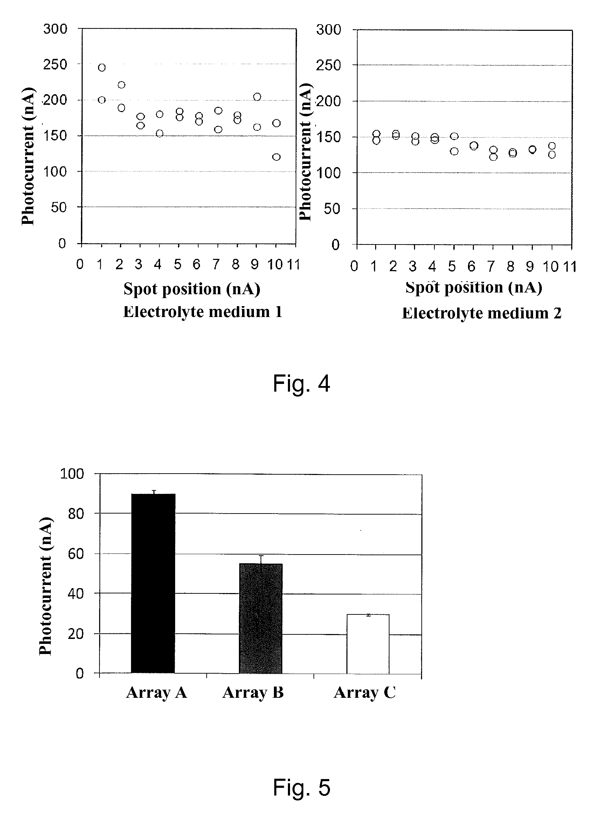

Subsequently, the electrolyte medium was charged into the sensor cell to fill inside the sensor cell; and then, the photocurrent was detected. Meanwhile, an acetonitrile solution (0.4 M of tetrapropyl ammonium iodide (NPr₄l, manufactured by Wako Pure Chemical Industries, Ltd.)) was used as the electrolyte medium 1. Separately from this, by using the working electrode fixed with the probe DNA which was prepared similarly, hybridization and washing were done exactly in the same procedures; and an aqueous solution (comprising 0.3 M of potassium ferrocyanide (manufactured by Wako Pure Chemical Industries, Ltd.), 0.01 M of potassium ferricyanide (manufactured by Wako Pure Chemical Industries, Ltd.), and 0.525 M of sodium chloride (manufactured by Wako Pure Chemical Industries, Ltd.)) was charged as the electrolyte medium 2 into the sensor cell to fill inside the sensor cell; and then, the photocurrent was detected. Results of the differences between the photocurrent values derived from respective spots and the base current values just before it in each line are shown in Fig. 4. As can be seen clearly, it was confirmed that by using the electrolyte medium 2, the dispersions among lines and rows were smaller so that the measurement could be done more accurately as compared with the case of using the electrolyte medium 1.

### Example 2

### Salt concentration of the aqueous washing solution:

Similarly to Example 1, the perfect match probe DNA was fixed as the probe DNA to the working electrode side, and the hybridization with the perfect match target DNA was carried out. Then, in order to wash out the excess target DNA and so forth not used in the hybridization, 1500 µL of aqueous washing solution 1 (3.5 x SCC) was charged into the sensor cell. Separately from this, by using the similarly prepared working electrode fixed with the probe DNA, each of the aqueous washing solutions having different salt concentrations, i.e., each of the aqueous washing solution 2 (1.5 x SSC) and the aqueous washing solution 3 (0.5 x SSC) was charged into the sensor cell. The ratios of the salt concentration of the binding reaction solution at the time of the hybridization reaction to the salt concentrations of the respective washing solutions were 7 for the aqueous washing solution 1, 2.33 for the aqueous washing solution B, and 1 for the aqueous washing solution 3, respectively. The fluorescence strengths of the respective working electrodes were measured at the exciting wavelength of 633 nm and the fluorescence wavelength of 670 nm by using the fluorescence scanner instrument Typhoon Trio (manufactured by GE Healthcare Life Sciences, Inc.); and the variation coefficients of the fluorescence values derived from the hybridization were calculated by using the image analysis software ImageQuant TL (manufactured by GE Healthcare Life Sciences, Inc.). As a result of it, they were 11.3% for the aqueous washing solution 1, 12.1 % for the aqueous washing solution 2, and 19.6% for the aqueous washing solution 3, respectively. From these, the results were obtained that the value and the variation coefficient were the smallest, and therefore, the effect thereof was the lowest, when the aqueous washing solution 1 was used among the aqueous washing solutions 1 to 3.

### Example 3

### Detection of single base change:

By the procedure similar to that of Example 1, 6 spots each of the perfect match probe (PM), the single base-changed chain (SNP), and the perfect mismatch probe (MM) were fixed to the working electrode side; and then, the single base polymorphism was detected by the apparatus and the detection method similar to those of Example 1. The respective base sequences were as following (same perfect match target DNA as Example 1 was used). As to the respective probes, the perfect match probe is shown by Array A, the single base-changed chain probe is shown by Array B, and the perfect mismatch probe is shown by Array C.
Perfect match probe: 5'-TGTAGGAGCTGCTGGTGCA-3' (SEQ ID No. 1)
Single base-changed chain (SNP) probe: 5'-TGTAGGAGCAGCTGGTGCA-3' (SEQ ID No. 5)
Perfect mismatch (MM) probe: 5'-TGAGCAAGTTCAGCCTGGT-3' (SEQ ID No. 6)

Results of the differences between the photocurrent values derived from respective spots and the base current values just before it with regard to the respective probe DNAs are summarized in Fig. 5. As can be seen clearly from the current values in Fig. 5, difference in one base (difference in the current values observed between PM and SNP) and perfect mismatch (difference in the current values observed between PM and MM) could be distinguished.

### Example 4

### Relationship between amount of the sensitizing dye and the photocurrent value:

By using the method of the present invention, relationship between amount of the sensitizing dye and the photocurrent value was confirmed. The perfect match probe DNA was fixed on the working electrode side as the probe DNA labeled with the sensitizing dye. The base sequence as following was used.

### Perfect match probe: 5'-TGTAGGAGCTGCTGGTGCA-3' (SEQ ID No. 1)

One molecule of the Cy5 dye is labeled relative to one molecule of DNA on the 5' terminal side. In addition, one amino group is labeled relative to one molecule of DNA on the 3' terminal side.

The coat glass (U film with the sheet resistivity of 12 Ω/square and the shape of 75 mm x 25 mm, manufactured by AGC Fabritech Co., Ltd.) having the fluorine-doped tin oxide (F-SnO₂: FTO) was arranged as the glass substrate for the working electrode. This glass substrate was cleaned by soaking in acetone for 15 minutes, and then by ultrapure water in the ultrasonic bath for 15 minutes to remove a dirt and an organic residue. This glass substrate was shaken in a 0.5-M aqueous sodium hydroxide solution for 15 minutes. Thereafter, to remove sodium hydroxide, it was shaken for cleaning in ultrapure water for 5 minutes; and this operation was repeated for 3 times by changing the water. The glass substrate was taken out, and after the residual water was blown off by air, the glass substrate was soaked in anhydrous methanol to remove water. 3-(Glycidyloxypropyl) trimethoxy silane (manufactured by Sigma-Aldrich Co.) was added to ultrapure water as the solvent such that the concentration thereof may become 2% by volume; and then, the mixture was stirred at room temperature for 5 minutes to obtain a coupling-treatment solution. The glass substrate was soaked in this coupling-treatment solution, and then, it was shaken slowly for 4 hours.

Next, the glass substrate was taken out and then shaken in ultrapure water for 3 minutes; and this operation was repeated for 3 times by changing water to carry out the operation to remove the excess coupling-treatment solution. Thereafter, the glass substrate was kept at 110°C for 30 minutes to bond the coupling agent to the glass substrate. After the glass substrate was cooled to room temperature, solutions of the probe DNA labeled with the sensitizing dye, having the concentrations in ultrapure water with 0.4, 0.2, 0.1, 0.08, 0.04, 0.02, 0.01, 0.008, 0.004, and 0 µM, respectively, were prepared; and then, after they were kept at 95°C for 10 minutes, they were immediately transferred onto an ice and kept there for 10 minutes to denature the DNA. These probe DNA solutions after denaturation (10 kinds) were spotted 2 each for every concentration in the short side direction with a total of 20 spots with 600 nL as the solution volume per 1 spot and with the distance between the spot centers being 5 mm by using the ink jet spotter Picojet 2000S with the nozzle of IJHBS-1000 (manufactured by Microjet Corp.). Thereafter, it was dried at 95°C for 3 minutes. The fluorescence intensities of the working electrode were measured at the exciting wavelength of 633 nm and the fluorescence wavelength of 670 nm by using the fluorescence scanner instrument Typhoon Trio (manufactured by GE Healthcare Life Sciences, Inc.); and the fluorescence values derived from the sensitizing dye of the probe DNA labeled with the sensitizing dye were calculated by using the image analysis software ImageQuant TL (manufactured by GE Healthcare Life Sciences, Inc.), thereby obtaining the calibration curve relative to the spotted amounts of the sensitizing dye.

Next, this working electrode was allowed to stand statically at 30°C and humidity of 90% for 24 hours to fix the probe DNA to the glass substrate. Washing by shaking in the 0.2% SDS solution for 5 minutes was repeated for 3 times; and then, washing by shaking in ultrapure water for 5 minutes was repeated for 3 times. After this glass substrate was soaked in boiling water for 2 minutes and taken out therefrom, the residual water was blown off by air. Subsequently, the glass substrate was soaked in anhydrous ethanol at 4°C for 1 minute to remove water, and then residual ethanol was blown off by air. By so doing, the working electrode fixed with the probe DNA labeled with the sensitizing dye was obtained.

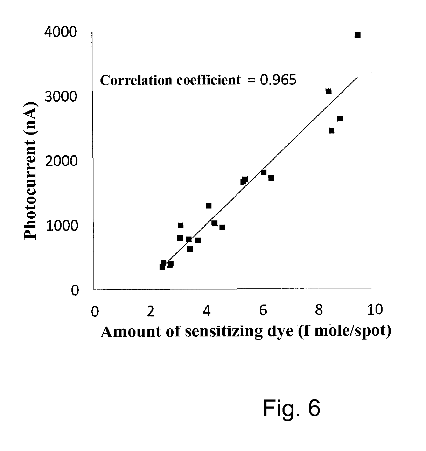

Similarly to Example 1, this electrode fixed with the probe DNA labeled with the sensitizing dye was installed as the working electrode inside the sensor cell 1 of the apparatus shown in Fig. 1. Subsequently, an aqueous solution (comprising 0.3 M of potassium ferrocyanide (manufactured by Wako Pure Chemical Industries, Ltd.), 0.01 M of potassium ferricyanide (manufactured by Wako Pure Chemical Industries, Ltd.), and 0.525 M of sodium chloride (manufactured by Wako Pure Chemical Industries, Ltd.)) was charged as the electrolyte medium into the sensor cell to fill inside the sensor cell; and then, the photocurrent was detected. Further, similarly to the above, the fluorescence intensity of the electrode fixed with the probe DNA labeled with by the sensitizing dye was measured by using the fluorescence scanner instrument; and then, the amount of the sensitizing dye present on the electrode fixed with the probe DNA labeled with the sensitizing dye was calculated by using the calibration curve that was previously calculated. The result of plotting of the calculated amounts of the sensitizing dye relative to the photocurrent values in the corresponding spots is shown in Fig. 6. As it can be clearly seen in Fig. 6, the amount of the sensitizing dye and the photocurrent value change almost linearly with the correlation coefficient of 0.965, so that it was confirmed that both are interrelated with each other.

### Example 5

### Electrolyte concentrations in various electrolyte media:

By using the method of the present invention, the studies were made on detection of the p53 gene and concentrations of various electrolyte media. The perfect match probe DNA was fixed to the working electrode side as the probe DNA. The base sequence thereof was as following.
Perfect match probe: 5'-TGTAGGAGCTGCTGGTGCA-3' (SEQ ID No. 1)

One amino group relative to one DNA molecule is labeled at the 3' terminal side.

The perfect match target DNA to be hybridized with this probe DNA was amplified by the PCR method. The base sequences of the primers and of the template DNA that were used in the amplification of the perfect match target DNA by PCR were as following.
Forward primer: 5'-Cy5-GATATTGAACAATGGTTCACTGAAGAC-3' (SEQ ID No. 2)
Reverse primer: 5'-GCCCCTCAGGGCAACTGAC-3' (SEQ ID No. 3)

Because multiplication is done by using the forward primer whose 5' terminal side is labeled with Cy5, one molecule of the Cy5 dye is labeled relative to one molecule of the target DNA.
Template DNA: 5'-GATATTGAACAATGGTTCACTGAAGACCCAGGTCCAGATGAAGCTCCCAGA ATGCCAGAGGCTGCTCCCCGCGTGGCCCCTGCACCAGCAGCTCCTACACC GGCGGCCCCTGCACCAGCCCCCTCCTGGCCCCTGTCATCTTCTGTCCCTT CCCAGAAAACCTACCAGGGCAGCTACGGTTTCCGTCTGGGCTTCTTGCATT CTGGGACAGCCAAGTCTGTGACTTGCACGGTCAGTTGCCCTGAGGGGC-3' (SEQ ID No. 4)

The coat glass (U film with the sheet resistivity of 12 Ω/square and the shape of 75 mm x 25 mm, manufactured by AGC Fabritech Co., Ltd.) having the fluorine-doped tin oxide (F-SnO₂: FTO) was arranged as the glass substrate for the working electrode. This glass substrate was cleaned by soaking in acetone for 15 minutes, and then by ultrapure water in the ultrasonic bath for 15 minutes to remove a dirt and an organic residue. This glass substrate was shaken in a 0.5-M aqueous sodium hydroxide solution for 15 minutes. Thereafter, to remove sodium hydroxide, it was shaken for cleaning in ultrapure water for 5 minutes; and this operation was repeated for 3 times by changing the water. The glass substrate was taken out, and after the residual water was blown off by air, the glass substrate was soaked in anhydrous methanol to remove water. 3-(Glycidyloxypropyl) trimethoxy silane (manufactured by Sigma-Aldrich Co.) was added to ultrapure water as the solvent such that the concentration thereof may become 2% by volume; and then, the mixture was stirred at room temperature for 5 minutes to obtain a coupling-treatment solution. The glass substrate was soaked in this coupling-treatment solution, and then, it was shaken slowly for 4 hours.

Next, the glass substrate was taken out and then shaken in ultrapure water for 3 minutes; and this operation was repeated for 3 times by changing water to carry out the operation to remove the excess coupling-treatment solution. Thereafter, the glass substrate was kept at 110°C for 30 minutes to bond the coupling agent to the glass substrate. After the glass substrate was cooled to room temperature, the probe DNA with the concentration of 10 µM in ultrapure water was prepared; and then, after it was kept at 95°C for 10 minutes, it was immediately transferred onto an ice and kept there for 10 minutes to denature the DNA. This probe DNA solution after denaturation was spotted 4 each in the short side direction with total 40 spots with 600 nL as the solution volume per 1 spot and with the distance between the spot centers being 5 mm by using the ink jet spotter Picojet 2000S with the nozzle of IJHBS-1000 (manufactured by Microjet Corp.). Thereafter, it was dried at 95°C for 3 minutes.

Next, this working electrode was allowed to stand statically at 30°C and humidity of 90% for 24 hours to fix the probe DNA to the glass substrate. Washing by shaking in the 0.2% SDS solution for 5 minutes was repeated for 3 times; and then, washing by shaking in ultrapure water for 5 minutes was repeated for 3 times. After this glass substrate was soaked in boiling water for 2 minutes and taken out therefrom, the residual water was blown off by air. Subsequently, the glass substrate was soaked in anhydrous ethanol at 4°C for 1 minute to remove water, and then residual ethanol was blown off by air. By so doing, the working electrode fixed with the probe DNA was obtained.

Next, 100 µL of an aqueous solution of the perfect match target DNA with the concentration thereof being adjusted 200 nM was mixed with 100 µL of an aqueous alkaline denaturing solution (2 mM of ethylenediamine tetraacetic acid (manufactured by Wako Pure Chemical Industries, Ltd.) and 0.2 M of sodium hydroxide (manufactured by Wako Pure Chemical Industries, Ltd.)); and then, the resulting mixture solution was kept at room temperature for 5 minutes to alkaline-denature the double chain DNA to the single chain DNA. To these solutions were added 700 µL of a dilute aqueous hybridization solution (5 x SSC, 0.1% SDS) and 100 µL of an aqueous neutralization solution (0.28 M hydrochloric acid (manufactured by Wako Pure Chemical Industries, Ltd.)), while temperatures of these solutions were adjusted at the prescribed hybridization temperatures; and then, they were mixed. This solution (1mL) after mixing was charged into the sensor cell, and then kept at 55°C in the sensor cell for 10 minutes to carry out the hybridization with the probe DNA fixed to the working electrode. In this occasion, before the hybridization solution was charged into the sensor cell, temperature inside the sensor cell was adjusted at the prescribed hybridization temperature by using the temperature controlling member comprised of a ceramic heater, a thermostat, and a thermistor. Ten minutes thereafter, the hybridization solution was completely discharged from inside the sensor cell by charging an air by using a pump. Then, to wash out the excess target DNA and so forth not used for the hybridization, 1500 µL of an aqueous washing solution (0.5 x SSC) was charged into the sensor cell.

Subsequently, the electrolyte media 3 to 7 as following were prepared as aqueous solutions by dissolving respective electrolytes into ultrapure water: electrolyte 3 (0.6, 0.3, 0.1, 0.05, and 0.01 M each of potassium ferrocyanide trihydrate (manufactured by Wako Pure Chemical Industries, Ltd.), 0.01 M of potassium ferricyanide (manufactured by Wako Pure Chemical Industries, Ltd.), and 0.525 M of sodium chloride (manufactured by Wako Pure Chemical Industries, Ltd.)); electrolyte 4 (0.6, 0.3, 0.1, 0.05, and 0.01 M each of ammonium ferrocyanide trihydrate (manufactured by Wako Pure Chemical Industries, Ltd.), 0.01 M of potassium ferricyanide, and 0.525 M of sodium chloride); electrolyte 5 (0.3, 0.1, 0.05, and 0.01 M each of hydroquinone (manufactured by Wako Pure Chemical Industries, Ltd.) and 0.525 M of sodium chloride); electrolyte 6 (0.1, 0.05, 0.01, 0.005, 0.001, and 0.0001 M each of L(+) ascorbic acid (manufactured by Wako Pure Chemical Industries, Ltd.) and 0.525 M of sodium chloride); and electrolyte 7 (0.9, 0.6, 0.3, 0.1, and 0.05 M each of potassium iodide (manufactured by Wako Pure Chemical Industries, Ltd.), 0.01 M of iodine (manufactured by Wako Pure Chemical Industries, Ltd.), and 0.525 M of sodium chloride). The respective aqueous solutions thereby prepared were charged into the sensor cell to fill inside the sensor cell; and then, the photocurrents were detected. These results are shown in Table 1. In each concentration of the respective electrolyte media, the photocurrents which were thought to be derived from the sensitizing dye of the hybridized target DNA molecule could be confirmed.

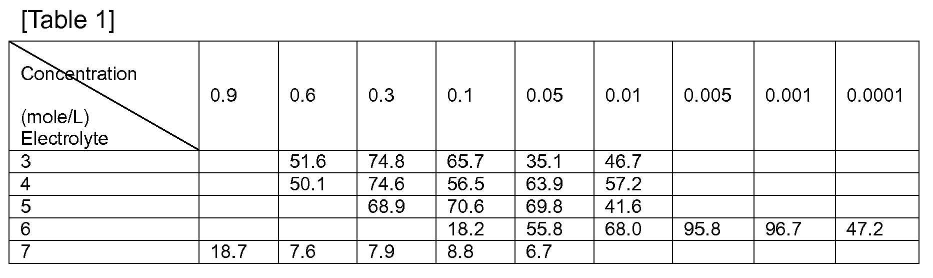

### Example 6

### Salt concentrations in various electrolyte media:

Similarly to Example 5, the working electrode fixed with the probe DNA using the perfect match probe was prepared, and then, hybridization thereof with 200 nM of the perfect match target DNA and the subsequent washing thereof were carried out.

Subsequently, respective electrolyte media 8 obtained as the aqueous solutions by dissolving respective electrolytes 8 (0.3 M of hydroquinone and 0.525, 0.2, 0.1, and 0.01 M each of sodium chloride) into ultrapure water were charged into the sensor cell to fulfill inside the sensor; and then, the photocurrents were detected. The results are shown in Table 2. In each salt concentration of the electrolyte media, the photocurrents which were thought to be derived from the sensitizing dye of the hybridized target DNA molecule could be confirmed.

**[Table 2]**

| | | | | |
|---|---|---|---|---|
| Salt concentration (M) | 0.525 | 0.2 | 0.1 | 0.01 |
| Salt concentration B of washing solution/Salt concentration C of electrolyte medium | 0.157 | 0.413 | 0.825 | 8.250 |
| Photocurrent value (nA) | 68.9 | 45.5 | 46.6 | 45.6 |

## Claims

1. A method for specific detection of a test substance, which comprises the steps of contacting both a working electrode having a sensitizing dye-labeled test substance fixed thereto via a probe substance and a counter electrode with an electrolyte medium, and then irradiating the working electrode with light to cause photoexcitation of the sensitizing dye, whereby detecting a photocurrent flowing between the working electrode and the counter electrode caused by electron transfer from the photoexcited sensitizing dye to the working electrode,
wherein
the working electrode has an electron-acceptor layer formed by containing an electron-acceptor substance capable of accepting an electron that is discharged in response to photoexcitation of the sensitizing dye, and the probe substance is attached on surface of this electron-acceptor layer;
a binding reaction step for contacting the working electrode to the test substance in the presence of a reaction solution to obtain a working electrode having the sensitizing dye-labeled test substance fixed thereto via the probe substance,
a washing step after the binding reaction step for washing out unbound substances with a washing solution, and
a measurement step in which the electrolyte medium is charged, and then the light is irradiated whereby measuring a photocurrent thus formed, are carried out in a single sensor cell; and
the electrolyte medium contains water and an electrolyte capable of donating an electron to the sensitizing dye in the oxidized state, while does not substantially contain an aprotic solvent.

2. The method for specific detection of a test substance according to claim 1, wherein
the reaction solution contains a salt with the concentration thereof in the reaction solution being represented by A, and
the washing solution contains a salt with the concentration thereof in the washing solution being represented by B; and based on this,
ratio A/B satisfies the relationship of 0.1≤A/B≤10 by mole ratio.

3. The method for specific detection of a test substance according to claim 1 or 2, wherein
the washing solution contains a salt with the concentration thereof in the washing solution being represented by B, and
the electrolyte medium contains a salt with the concentration thereof in the electrolyte medium is represented by C; and based on this,
ratio B/C satisfies the relationship of 0.1≤B/C≤100 by mole ratio.

4. The method for specific detection of a test substance according to any one of claims 1 to 3, wherein the test substance is a biomolecule.

5. The method for specific detection of a test substance according to any one of claims 1 to 4, wherein the probe substance is a biomolecule.

6. The method for specific detection of a test substance according to claim 4 or 5, wherein the biomolecule is DNA.

7. The method for specific detection of a test substance according to any one of claims 2 to 6, wherein the salt comprising sodium chloride.

8. The method for specific detection of a test substance according to any one of claims 1 to 7, wherein the electrolyte comprises ferrocyanide salt.

9. The method for specific detection of a test substance according to claim 8, wherein the ferrocyanide salt comprises potassium ferrocyanide.

10. The method for specific detection of a test substance according to claim 8 or 9, wherein the ferrocyanide salt comprises ammonium ferrocyanide.

11. The method for specific detection of a test substance according to any one of claims 1 to 10, wherein the electrolyte comprises hydroquinone.

12. The method for specific detection of a test substance according to any one of claims 1 to 11, wherein the electrolyte comprises L-ascorbic acid.
